(19) Europäisches Patentamt
European Patent Office
Office européen des brevets

(11) **EP 4 345 836 A1**

(12) **EUROPEAN PATENT APPLICATION**

(43) Date of publication:
**03.04.2024 Bulletin 2024/14**

(21) Application number: **22198895.9**

(22) Date of filing: **29.09.2022**

(51) International Patent Classification (IPC):
**G16H 30/40** (2018.01) **G16H 50/50** (2018.01)

(52) Cooperative Patent Classification (CPC):
**G16H 30/40; G16H 50/50**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Kardiolytics Inc.**
**Tulsa, OK 74114 (US)**

(72) Inventors:
• **SIEMIONOW, Kris**
  **Chicago (US)**
• **LEWICKI, Paul**
  **Tulsa (US)**
• **MALOTA, Zbigniew**
  **Tychy (PL)**
• **SADOWSKI, Wojciech**
  **Zabrze (PL)**

(74) Representative: **Kancelaria Eupatent.pl Sp. z.o.o**
**Ul. Kilinskiego 185**
**90-348 Lodz (PL)**

(54) **A METHOD AND SYSTEM FOR DETERMINING INFLUENCE OF BIOMECHANICAL FORCES ON DEFORMATION AND STRESSES OF ARTHROSCLEROSIS PLAQUE**

(57) A computer-implemented method for modelling blood vessels to support assessment of probability of rupture or damage to the plaque. The method includes steps of: obtaining medical imaging data of the blood vessels; generating a three-dimensional model of the blood vessels, based on the medical imaging data including identifying one or more pathological plaques; performing pre-simulation of the three-dimensional model, establishing boundary conditions and initial conditions for both models for a steady flow of blood and a transient flow of blood; performing a numerical simulation of the transient flow of blood; performing a numerical simulation of the steady flow of blood; and for a selected plaque, identifying geometrical parameters of a surface of the plaque, including shape, curvature, curvature of the major surface, and/or Gauss curvature of the plaque surface. The method may include calculation of Reference Dynamic Pressure (RDP) and Degree of Stenosis (DS).

Fig. 1

EP 4 345 836 A1

**Description**

**TECHNICAL FIELD**

The present invention relates to modelling blood vessels and determining influence of biomechanical forces on deformation and stresses of arthrosclerosis plaque, in particular to predict probabilities of rupture or damage to the atherosclerotic plaque.

**BACKGROUND**

Atherosclerotic plaques develop in specific areas of the coronary arteries in which the flow is disturbed, most often on the outer side walls of the bifurcation and on the internal curvature of the arteries. (Bentzon JF, 2014). Hemodynamic factors such as shear stress wall, oscillatory shear index - OSI, relative residence time - RRT, fractional flow reserve - FFR or pressure drop factor - CPD are important from the point of view of hemodynamic significance of stenosis and atherosclerotic plaques progression (Malota, 2018). Areas of low-level WSS that are associated with high OSI or RRT have a high risk of atherosclerotic plaque formation, while high WSS values in already narrowed, atherosclerotic plaques promote the atherosclerotic plaque rupture by destabilizing the fibrous cap (Brenda R.Kwak et al., 2014), (Vikas Thondapu et al., 2017). A large part of acute coronary events is the result of sudden vessel thrombosis due to erosion or rupture of the atherosclerotic plaque. Atherosclerotic plaque rupture occurs when the cap is the thinnest and most infiltrated by foam cells (macrophages). In eccentric plaques, the weakest point is often the edge of the cap, and only extremely thin fibrous caps are at risk of rupture. The morphological characterization of the sensitive coronary plaques has been well defined in numerous pathological studies (Bentzon J.B., 2014), (Adam J. Brown et al., 2016; Malek AM, Alper SL, Izumo S, 1999).

Typically, such plaques have a large extracellular necrotic lipid core, areas of calcification (plaque haemorrhage / thrombus PH / T) and a fibrous cap (FC) of less than 65 □m thickness. Although in recent years the knowledge about the sensitive features of atherosclerotic plaque has significantly expanded, the prediction of susceptible coronary artery rupture is still imprecise.

The mechanism of plaque formation is very complex (Bentzon J.B., 2014). In the first stage of atherosclerotic plaque formation, the stable fibromuscular changes appears, mainly containing fibrous tissue with minimal areas of calcification and small lipid pools. In the last stage, the fully formed plaque, vulnerable to rupture and damage, contain a large stiff necrotic spine, with a large lipid-rich core, with areas of calcium and inflammatory cells closed with a thin about 65 m thickness fibrous cap. The mechanical properties of the vessel and plaque depend on the stage of atherosclerosis (C.Akyildiz, 2014).

Coronary arteries are subject to continuous biomechanical forces action during each heart cycle. This forces are working in the peripheral, radial and axial directions and deforms the artery wall creating stresses in them. Axial stress arises as a result of longitudinal stretching of the vessel during varying flow rates during the cycle. It is believed that axial stresses practically they have much less impact on the formation of atherosclerosis than radial and peripheral stresses. (Vikas Thondapu et al., 2017). However, in a vessel with already formed atherosclerotic plaque, it can affect its damage.

The main biomechanical factors affecting the susceptibility and mechanical instability of the atherosclerotic plaque are: its geometry, composition and hemodynamic stresses (Finet, 2007). In particular, the low and oscillatory shear stresses, which activate endothelial cells leading to the accumulation of macrophages in the vascular membrane (Seneviratne A., 2013).

Endothelial cells are extremely sensitive to shear stress, and the frictional forces generated by blood flow. The mean shear stress of the coronary vessel wall varies from 1-2 Pa (Doriot PA, 2000) and the peripheral stress varies from 100 to 200 kPa , depending on the anatomical site.

In the areas of arterial stenosis in accordance with Bernoulli's law, the speed of blood flow and the wall shear stresses increases, while the pressure decreases, and thus the radial and circumferential stresses decrease to. The opposite situation is observed directly behind the stenosis where the average velocity of the flow stream and the wall shear stress decreases, while the static pressure increases. There is a negative flow and negative pressure gradient in a separated swirl layer. This way, the endothelium below the stenosis is exposed to the tangential stress oscillations. On the other hand, radial and circumferential stresses are greater.

In scientific publications, the importance of two types of stress affecting atherosclerosis, i.e. wall shear shear stress (WSS) and the plaque structural stress (PSS) is widely emphasized. The influence of WSS on atherosclerotic processes depends on its level (Malek AM, Alper SL, Izumo S, 1999).

PSS is a stress induced inside the atherosclerotic plaque or the arterial wall as a result of stretching the vessel by the arterial pressure. PSS is also determined in part by the morphology of platelets and by tissue material properties. High levels of PSS may be the main mechanism of plaque rupture, leading to thrombosis and sudden ischemic clinical events. In a vessel with uniform thickness and wall pressure, higher PSS values are observed in the proximal coronary segments. In contrast, lower PSS values can be expected at the stenoses and with the increasing thickness of the vessel wall. However, changes in the composition and structure of the artery wall and plaque can significantly change the PSS.

In recent years, several publications confirmed the potential impact of axial forces induced by the flow stream, in atherosclerotic plaque rupture. (axial plaque stress (APS)). (Choi G., 2015).

When blood flows through a constricted blood vessel, pressure and kinetic energy are converted (ignoring gravity), but some energy is lost mainly by friction. The energy losses take one of three forms: viscous losses, turbulence losses, and flow separation losses. Viscous energy losses are proportional to flow rate. Turbulent and separated losses are in general proportional to the square of the flow rate. Turbulent losses usually do not occur in the healthy vessel but in severe stenosis they can be of greater magnitude than viscous losses.

The mechanical energy flow rate EF expressed in Watt, which is needed to overcome the (constant) flow resistance and maintain the specified blood flow through cross sectional area of the coronary artery is represented as the product of the total pressure $P_{total}$ and the mass flow rate Q (assuming no gravitational effects):

$$EF_i = \left(\frac{1}{2}\rho u^2 + P\right)_i Q_i = (P_{total})_i\, Q_i\ [W]$$

When blood passes through the stenosis results in an additional, local total pressure drop according to Bernoulli's equation for real liquids.

The total energy (E) of the blood flowing within the vessel, therefore, is the sum of the kinetic and potential energies as expressed by Bernoulli's principle.

This Dynamic pressure is proportionate to the mean velocity squared. At the point of narrowing, it increases sharply (with a decrease in static pressure) and represents the force acting directly on the plate over its entire area.

The accurate measurement of coronary stresses is still a challenge, but advanced computational techniques allow on numerical analysis of its distribution and size.

Due to the complex shape of the vessels and the plaque itself, the description of the forces acting directly on the plaque, can be important additional information on the risk of plaque rupture and may help identify sensitive sites of stenoses.

Numerical analysis of the shape and deformation of the cap (bulging) (Abdelali M, 2014) also allows determining the sites of probable atherosclerotic plaque damage.

SUMMARY

The inventors have carried out research focused on the analysis of the influence of biomechanical forces, generated by the blood flow in the coronary vessel, on the deformation of the plaque surface and the stress acting on it. According to the results presented in (Costopoulos C, 2019) , (Kwak BR, 2014):

- Low WSS <0.4 Pa- are susceptible to atherogenesis and plaque lesion formation which may promote inflammation and affect the stability of atherosclerotic plaque;
- Average Supraphysiological WSS <10 Pa,> 2 Pa, above the normal values found in the vessels, WSS may be the causes of plaque rupture or clots (thrombus) generation;
- High WSS> 10 Pa directly do not affect the development of atherosclerosis but may cause thinning of the plaque wall and its destabilization (atheroprotection)

While, high stress located inside the body of an atherosclerotic plaque or the arterial wall as a consequence of vessel expansion and stretch induced by arterial pressure , plaque structural stress(PSS), caused fibrous cap damage, for example, rupture, periodic change of PSS: fibrous cap fatigue.

The purpose of this invention is to solve problems that occur in known methods of supplementing information on cardiovascular diseases, the results of which are intended for medical practitioners and which can be used in further diagnostics. Essentially, the analysis of blood flow dynamics in generated models reflecting blood vessels itself is very difficult mainly due to complex geometry of the blood vessels, the flexibility of their walls, non-Newtonian properties of blood, pulsatile flow and variable vascular resistance, during the diastolic and systolic phases of the cardiac cycle. Particular difficulties are related to both the lack of detailed clinical data as well as individual (personalized) differences in cardiovascular physiology enabling precise mathematical and physical models generation.

Atherosclerotic plaques are subject to continuous biomechanical forces action and are a hydraulic obstacle to blood flow. The flow resistance through the constricted vessel depends on both the degree of constriction and its location. The blood flow stream exerts pressure, a force acting on an obstruction (stenosis), which causes stresses both on the surface and inside the plaques.

This forces are working in the peripheral, radial and axial directions and deforms the artery wall creating stresses in them. Axial stress arises as a result of longitudinal stretching of the vessel during varying flow rates during the cycle. It is believed that axial stresses practically they have much less impact on the formation of atherosclerosis than radial and peripheral stresses (Vikas Thondapu et al., 2017). However, in a vessel with already formed atherosclerotic plaque, it can affect its damage.

The main biomechanical factors affecting the susceptibility and mechanical instability of the atherosclerotic plaque are: its geometry, composition and hemodynamic stresses (Finet, 2007). In particular, the low and oscillatory shear stresses, which activate endothelial cells leading to the accumulation of macrophages in the vascular membrane (Seneviratne A., 2013).

Endothelial cells are extremely sensitive to shear stress, and the frictional forces generated by blood flow. The mean shear stress of the coronary vessel wall varies from 1-2 Pa (Doriot PA, 2000) and the peripheral stress varies from 100 to 200 kPa , depending on the anatomical site.

In the areas of arterial stenosis in accordance with Bernoulli's law, the speed of blood flow and the wall shear stresses increases, while the pressure decreases, and thus the radial and circumferential stresses decrease. The opposite situation is observed directly behind the stenosis where the average velocity of the flow stream and the wall shear stress decreases, while the static pressure increases.

There is a negative flow and negative pressure gradient in a separated swirl layer. This way, the endothelium below the stenosis is exposed to the tangential stress oscillations. On the other hand, radial and circumferential stresses are greater.

It is very difficult to determine the stress values due to very complex structural properties (Cheng GC, 1993), (Loree HM, 1992), (Lee RT, 1991).

Therefore, the assessment of the risk of plaque rupture usually should include the analysis of the stress state of the atherosclerotic plaque should include the analysis of blood flow dynamics - CFD (Computational Fluid Mechanics) and the structural analysis of plaque deformation - CSM (Computational Solid Mechanics).

The invention presented herein makes it possible to assess the risk of atherosclerosis rupture without a comprehensive structural analysis.

The object of the invention is a computer-implemented method for modelling blood vessels, the method comprising the steps of: obtaining medical imaging data of the blood vessels; generating a three-dimensional model of the blood vessels, based on the medical imaging data including identifying one or more pathological plaques; performing pre-simulation of the three-dimensional model, establishing boundary conditions and initial conditions for both models for a steady flow of blood and a transient flow of blood; performing a numerical simulation of the transient flow of blood, for selected physical and boundary conditions, for the three-dimensional model for an increasing blood flow rate that increases from a laminar flow to a developed turbulent flow, the simulation comprising, determined during the pre-simulation, initial conditions of blood flow; performing a numerical simulation of the steady flow of blood, for selected physical and boundary conditions, for the three-dimensional model for transitional or turbulent flow, the simulation comprising, determined during the pre-simulation, initial conditions of blood flow; and for a selected plaque, identifying geometrical parameters of a surface of the plaque, including shape, curvature, curvature of the major surface, and/or Gauss curvature of the plaque surface.

The method may further comprise determining a pressure force acting on the plaque surface at any one or more points as a force acting on a fixed curved wall of the atherosclerotic plaque for a steady flow and/or for a transient flow, preferably by means of a formula:

$$F = \sqrt{F_T^2 + F_N^2} = 2\rho Q U sin\frac{\alpha}{2}.$$

wherein:

$\alpha$ is the angle of the slope between the tangent curve at the pressure point and the main axis of the vessel;
$F_T$ and $F_N$ are tangent and normal components of force to the surface of the plaque at the point of action;

The method may further comprise determining a probability of rupture or damage to the plaque, by comparing the pressure force present in the plaque with a reference pressure force for normal, healthy coronary vessels, by a calculated Reference Dynamic Pressure (RDP) and Degree of Stenosis (DS), wherein the Reference Dynamic Pressure (RDP) expresses the ratio of the dynamic pressure in the stenosed model to the dynamic pressure in the reference model in the plaques region and the Degree of Stenosis (DS) is a coefficient that increases as a function of the degree of vessel constriction.

The invention also relates to a computer-implemented system, comprising: at least one nontransitory processor-readable storage medium that stores at least one of processor-executable instructions or data; and at least one processor communicably coupled to at least one nontransitory processor-readable storage medium, wherein at least one processor is configured to perform the steps of the method as described herein.

BRIEF DESCRIPTION OF DRAWINGS

Various embodiments are herein described, by way of example only, with reference to the accompanying drawings, wherein:

Fig. 1 shows dynamic forces of a flow stream acting on an atherosclerotic plaque mode;
Fig. 2 shows definition of main curvatures;
Fig. 3 shows measurement points of Gauss curvature and selected plaques curvatures;
Fig. 4 shows an example of a numerical model of atherosclerosis plaque;
Fig. 5 shows a cross-section curve of the atherosclerotic plaque along the vessel axis with marked measurement points, values of the plaque geometry curvature along the vessel axis and Gaussian curvature determined on the basis of 5 measurement points;
Fig. 6 shows Von Mises Stress along the vessel axis inside fibrous cap;
Fig. 7 shows stress distribution and Gauss curvature over time at five measuring points;
Fig. 8 shows influence of curvature, angle of inclination of a tangent curve to axis of vessel on the axial flow and pressure force;
Fig. 9 shows results of numerical simulation;
Fig. 10 shows total, static and dynamic pressure changes in an axis of schematic vessel stenosis with a conical collector and sudden expansion;
Fig. 11 shows an example plot of a risk of rupture or damage to the atherosclerotic plaque;
Fig. 12 shows a method of the invention; and
Fig. 13 shows a computer-implemented system for performing the method.

DETAILED DESCRIPTION

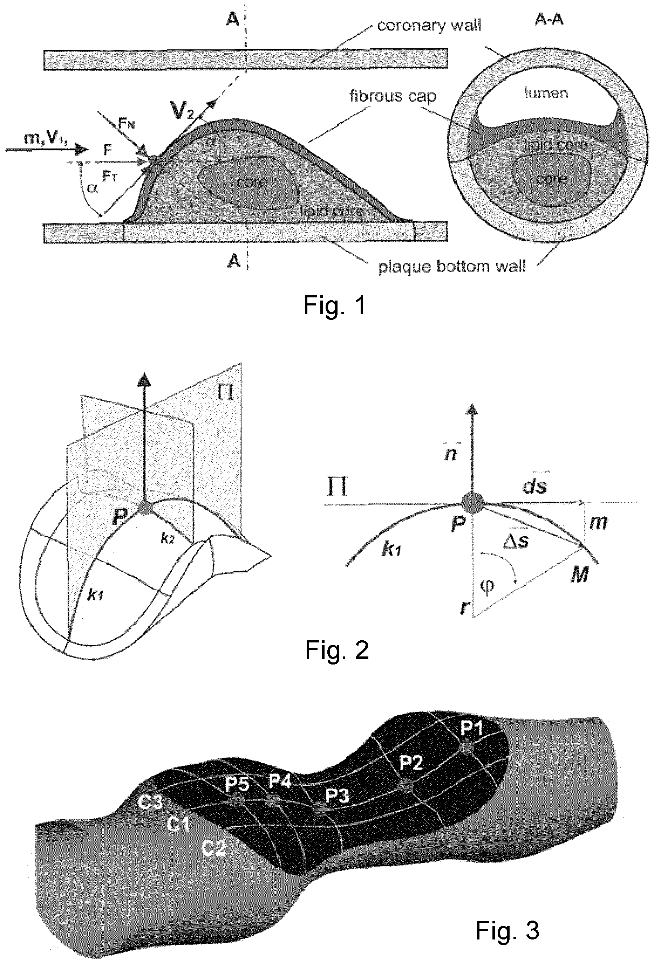

The following detailed description is of the best currently contemplated modes of carrying out the invention. The description is not to be taken in a limiting sense, but is made merely for the purpose of illustrating the general principles of the invention.
The analysis is based on new parameters related to the curvature of the lamina surface and the external force acting on it, as shown in Fig. 1.
The force resulting from the action of the blood stream on the plaque i.e dynamic pressure can be estimated from the principle of conservation of momentum.
In the case of a free stream acting tangentially on a fixed curved wall of the atherosclerotic plaque, the force of reaction can be calculated from the formulas:

$$F = \sqrt{F_T^2 + F_N^2} = 2\rho QU sin\frac{\alpha}{2}.$$

wherein:

$\alpha$ is the angle of the slope between the tangent curve at the pressure point and the main axis of the vessel;
$F_T$ and $F_N$ are tangent and normal components of force to the surface of the plaque at the point of action.

$$F_T = \rho QU(1 - cos\alpha),$$

$$F_N = -\rho QU sin\alpha,$$

The force of reaction depends on the angle of incidence, i.e. the shape of the plate. The angle of incidence can be defined as the angle between flow stream incident on a surface and the line perpendicular (normal) to the surface at the point of incidence. The shape of the plaque surface can be described by curves.
Typically, such plaques have a large extracellular necrotic lipid core, areas of calcification (plaque haemorrhage / thromous PH / T) and a fibrous cap (FC) of less than 65 □m thickness. Although in recent years the knowledge about the sensitive features of atherosclerotic plaque has significantly expanded, the prediction of susceptible coronary artery rupture is still imprecise.

The shape of the surface has been characterized in the appropriate coordinate system by means of two main curvatures on the basis of which the Gaussian curvatures were determined. The local curvature of Gauss is the product of main curvatures intersecting at the point of surface where force acting. The main curves were defined as (see Figs. 2, 3):

$$k = \lim_{\Delta S \to 0} \frac{\Delta\varphi}{\Delta S} = \frac{d\varphi}{dS}$$

where $\Delta\varphi$ is the angle between tangents to the curve at the ends of the curve, and $\Delta S$ is the length of the arc.

The straight line has a curvature = 0, the curvature of the circle is inversely proportional to its radius, while the Gauss curvature of both flat and cylindrical surface is 0, in the case of the sphere the Gauss curvature is 1 / r2.

It should be noted that the Gaussian curvature is an invariant of isometry. If we deform a curvature without stretching or compression, the Gauss curvature does not change. However, the mean curvature of the surface changes, which is the sum of the main curvatures. The change in Gauss curvature as an indicator of plate deformation is therefore directly related to the stress action. The magnitude of the thrust force also depends on the value of this curvature. The greater the curvature, the greater the stresses are generated. In the place of the greatest pressure, a deformation (cavity) of the lamina occurs with simultaneous bulges on both sides of the cavity. Such buckling of the plaque shell (cap) is considered a potential destabilizing factor that increases the tendency of the plaque to rupture and which may also explain plaque damage away from the hat caps (Abdelali M, 2014). The greater the bulge, the greater the curvature of the plaque and the greater the likelihood of it rupture or damage. The change in the curvature depends on the pressure force, i.e. the flow rate and the angle of incidence.

When the shell bulges, the inflection points appears, i.e. there is a change in the sign of curvature. The more inflection points, the more buckling, unevenness and the greater the probability of the plate breaking. The greatest stresses appears near the tip of the plaque where angle of surface curvature is greatest. In particular, there is a great risk when the curvature changes from concave to convex with respect to the flow direction - then the greatest angle of inclination of the plaque surface is observed.

Generally , when blood flows through a constricted blood vessel, pressure and kinetic energy are converted (ignoring gravity), but some energy is lost mainly by friction. Frictional energy losses take one of three forms: viscous losses, turbulence losses, and flow separation losses. Viscous energy losses are proportional to flow rate. Turbulent and separated losses are in general proportional to the square of the flow rate. Turbulent losses usually do not occur in the healthy vessel but in severe stenosis they can be of greater magnitude than viscous losses. Flow separation occurs when flow has experienced a sudden expansion causing a decrease in kinetic energy and rise in pressure. The rise in pressure occurs can overcome the inertia near the vessel wall, reversing its direction. The flow separation losses represent energy necessary to reinitiate the forward movement of the fluid.

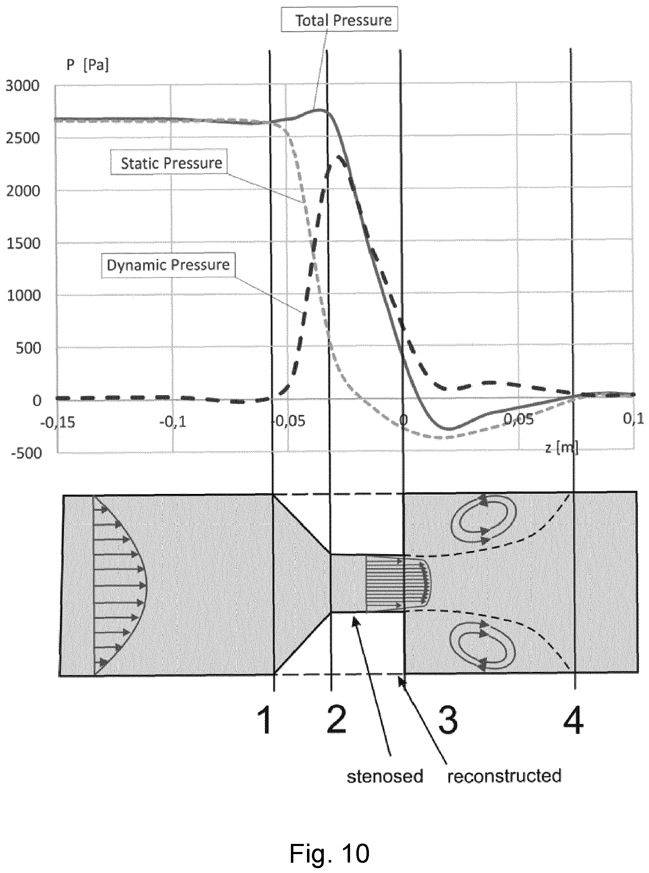

The area across a hypothetical arterial stenosis can be divided into contraction, throat, and expansion sections, as shown in Fig. 10.

It should be noted, there is a spatial offset along stenosis between the static pressure and the total pressure , that increases in magnitude with increased degree and length of stenosis. The greatest static pressure drop, caused by stenosis, is mainly observed in the initial region of stenosis, (contraction section), despite a small drop in total energy. Whereas, in the final area of the constriction, (expansion section) and immediately downstream to stenosis, a large amount of energy is lost, associated mainly with the losses of kinetic energy, despite, according to Bernoulli's equation, the increase in static pressure (pressure recovery phenomenon).

The dynamic pressure losses of the flow energy, which occur mainly at the site of the stenosis, represent the total force acting directly on the plaque throughout its entire area. It can therefore also be an additional new parameter determining the probability of plaque damage, taking into account changes in dynamic pressure during blood flow through the stenosed vessel compared to the dynamic pressure in a healthy (reference) vessel.

The force F of the reaction on atherosclerotic plaque can be expressed by dynamic pressure.

$$F = 2\rho QU sin\frac{\alpha}{2} = 2\rho U^2 S sin\frac{\alpha}{2} = 4P_d S sin\frac{\alpha}{2}$$

wherein:

$P_d$ is a dynamic pressure (average value of the cross-sectional area normal to the vessel axis or value at the vessel centerline), $P_d = \dfrac{\rho U^2}{2}$ ,

S is a cross-section of stenosis.

The values of the above parameters can be calculated at any point of stenosis (e.g. at the point of maximum constriction) or averaged over the entire length of the stenosis

The use of the comparative method, in which relative indices are determined on the basis of a comparison of two models, narrowed and reconstructed, of the same patient, allows to determine the coefficients determining the risk of rupture or damage to the atherosclerotic plaque.

In the case of a specific flow (due to autoregulation of coronary flow), the same in both the reference model and model with the narrowing, the reduction of the active cross-section due to the development of the lamina causes an increase in the flow velocity in the narrowing area, i.e. an increase in the dynamic pressure

The comparison of the maximum values of the forces occurring in the model with the constriction (s) with the values occurring in the reference model (r) acting on the same hypothetical obstacle can be written as:

$$\frac{F_s}{F_r} \approx \frac{(P_d S)_s}{(P_d S)_r} = \frac{(P_d)_s}{(P_d)_r} * \frac{(S)_s}{(S)_r} = \frac{1 - DS}{RDP}$$

wherein:

RDP is a relative dynamic pressure coefficient expresses the ratio of the dynamic pressure in the stenosed model to the dynamic pressure in the reference model in the plaques region,

$$RDP = \frac{(P_d)_r}{(P_d)_s}.$$

In case of no stenosis, RDP = 1. The greater the value of the dynamic pressure at the area of the plaque, the lower the RDP value,

DS is a degree of stenosis, $DS = 1 - \dfrac{(S)_s}{(S)_r}$

In case of no stenosis, DS = 0. In case of vessel occlusion, DS = 1.

Alternatively, a value of the Reference Dynamic Pressure (RDP) multiplied by the Degree of Stenosis (DS) may be analyzed, or another function based on the RDP and DS values.

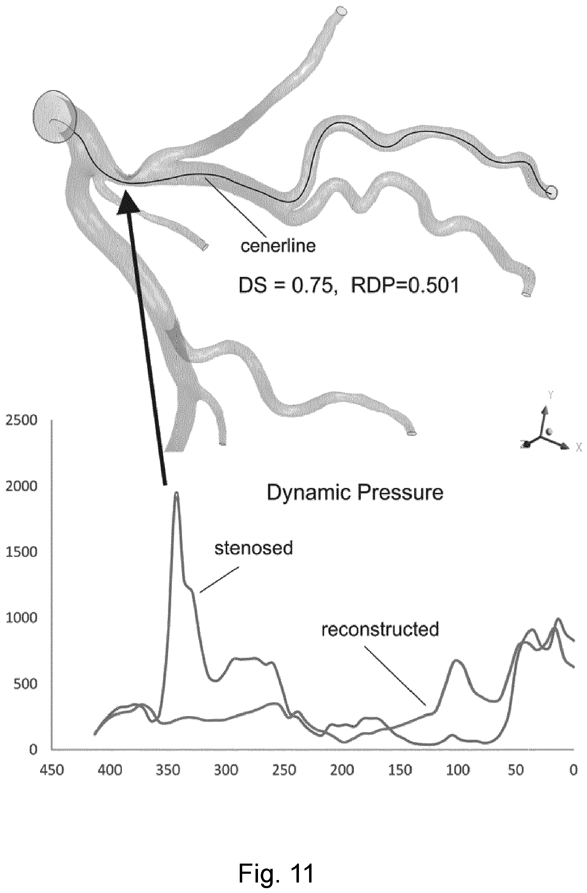

Fig. 11 shows an example plot of a risk of rupture or damage to the atherosclerotic plaque on an example of the left coronary artery with a stenosis (~ 75%) in the proximal section of the left circumferential branch (CX). The plaque rupture or damage index is calculated as the ratio of the dynamic pressure multiplied by the stricture area of the reconstructed and stenosed models.

The method of modelling blood vessels and blood flow in these blood vessels models allows to determine various information on blood flow, using for this purpose only non-invasively collected patient information. The modelling method may refer to blood flow in any blood vessels, such as coronary, cervical, peripheral vessels, abdominal cavities, kidneys and cerebral vessels.

The curves are measured at five characteristic points lying on the surface of the plaque. To analyze the curvature, software such as Matlab R2015b can be used.

It is assumed that the deformation of plaques under the influence of force acting on them is proportional to this force. The ratio between the stress caused by the applied forces and the deformation is described by the stress tensor. For describing the state of the complex atherosclerotic plaque stress condition, the strain tensor and reduced von Misses stress are applied (Mises., 1913), (Jones, 2009).

A full numerical analysis of atherosclerotic plaque should take into account the analysis of the dynamics of blood flow - CFD (Computational Fluid Mechanics) and structural analysis of the deformation of the plaque - CSM (Computational Solid Mechanics).

For this reason, a coupled Fluid Strutured Interaction method (FSI) based on Newtonian blood model and Shear Stress Transport (SST) turbulence model is used. This model allows the analysis of the interaction of the blood stream with specific mechanical properties of artery wall (Menter, 1994).

The numerical calculations can be performed using a software package such as ANSYS Multiphysics.

The structural properties of the plaque are very complex (Cheng GC, 1993), (Loree HM, 1992), (Lee RT, 1991). However, an isotropic model of all the elements of the plate and the wall of the coronary artery were accepted. A constant elastic modulus of 100 KPa is assumed for the artery. The modulus of elasticity of the plaque elements can be various, in the range from 1 kPa to 10,000 kPa. This allows the plaque to be analyzed at various stages of its development, in which the core module is smaller, equal to and larger than the modulus of elasticity of the upper cap.

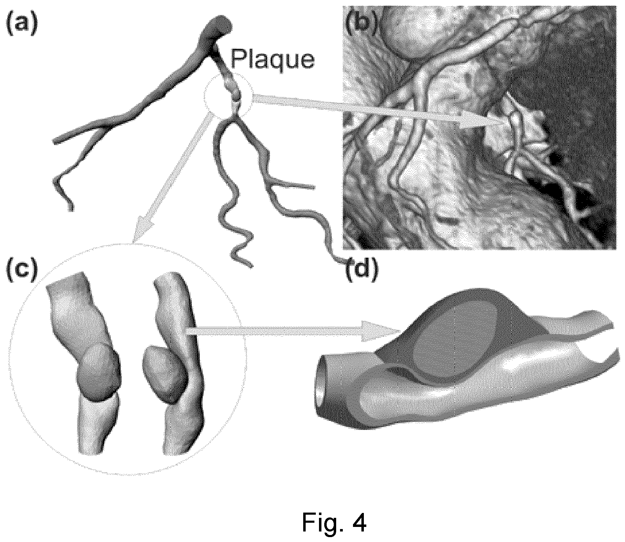

The atherosclerotic plaque geometry can be obtained based on the segmentation of images CT of Left main coronary artery LMCA, for example by using the 3D-DOCTOR (Able Software Corp.) and 3DSlicer software package. Fig. 4 shows an example of a numerical model of atherosclerosis plaque. The finite element mesh FEM in the present example contains approx. 1.2 million tetragonal elements. The simulation is carried out for a pulsed flow with waveform of pulse was appropriate for the inlet left coronary artery and with an average flow rate of 0.3 l / min. ang heart rate of 60 Hz.

The force resulting from the action of the blood stream on the plaque, i.e. the dynamic pressure can be estimated from the principle of conservation of momentum. In the case of a free stream acting tangentially on a fixed curved wall of the atherosclerotic plaque, the force of reaction can be calculated from the formulas:

$$F = \sqrt{F_T^2 + F_N^2} = 2\rho QU sin\frac{\alpha}{2}.$$

wherein:

$\alpha$ is the angle of the slope between the tangent curve at the pressure point and the main axis of the vessel,
$F_T$ and $F_N$ are tangent and normal components of force to the surface of the plaque at the point of action.

The curvature of the plaque in its central part has the opposite sign to the curvature in the front and the end part. The section profile of the plate along the axis of the artery usually has at least two points of inflection for which the curvature is 0. However, the value of the curvature also depends on the stages of plaque development. The surface of the plaque, and thus its main curvature, is deformed during the entire heart cycle. The largest change in the curvature is observed in the initial stages of plaque formation when the modulus value of core elastic is comparable to the modulus value of liquid core.

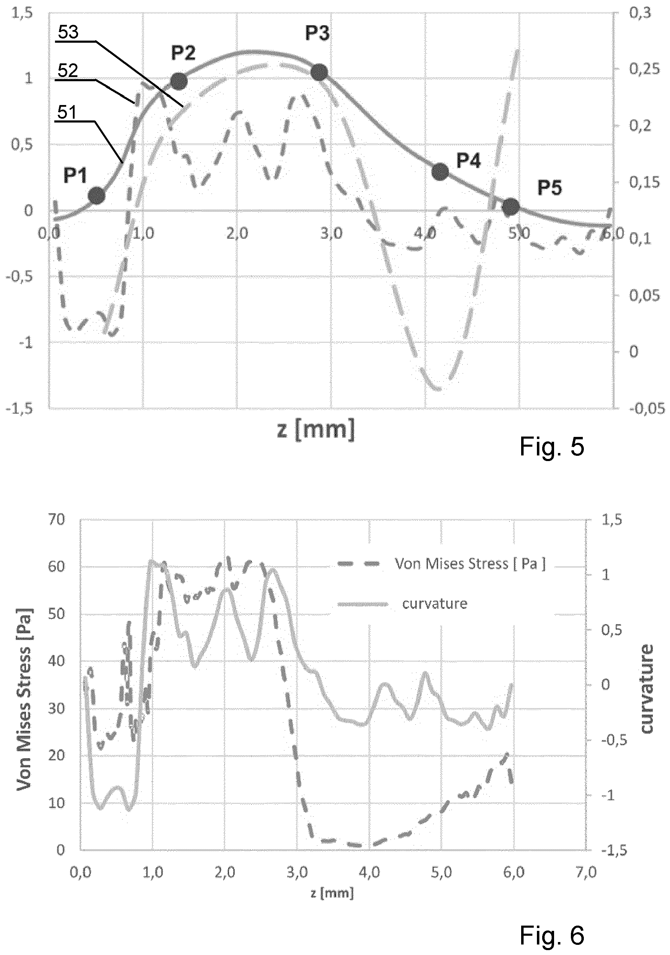

Fig. 5 shows a cross-section curve 51 of the atherosclerotic plaque along the vessel axis with marked measurement points, values 52 of the plaque geometry curvature along the vessel axis and Gaussian curvature 53 determined on the basis of 5 measurement points P1-P5.

Fig. 6 shows Von Mises Stress along the vessel axis inside fibrous cap.

As shown in Figs. 5 and 6, the distribution of stresses generated inside the fibrous cup is consistent with the distribution of the curvature of the lamina surface. The maximum intensities are observed in the places of maximum curvature at the peak of the plaque and below near place of inflection points of the curvature. Therefore, the curvature analysis can also help to assess the state of stresses and deformations in the plaque as well can be useful tools for determine the probability of the risk of plaques rupture and localization of the places most vulnerable on the rupture or damage.

Such a fast, non-invasive analysis of the plaque shape together with the pressure force analysis, without the need to apply complex structural calculations, will allow at any stage of plaque development to assess the risk of plaque rupture and damage resulting from the hemodynamic forces and assess the need for medical intervention.

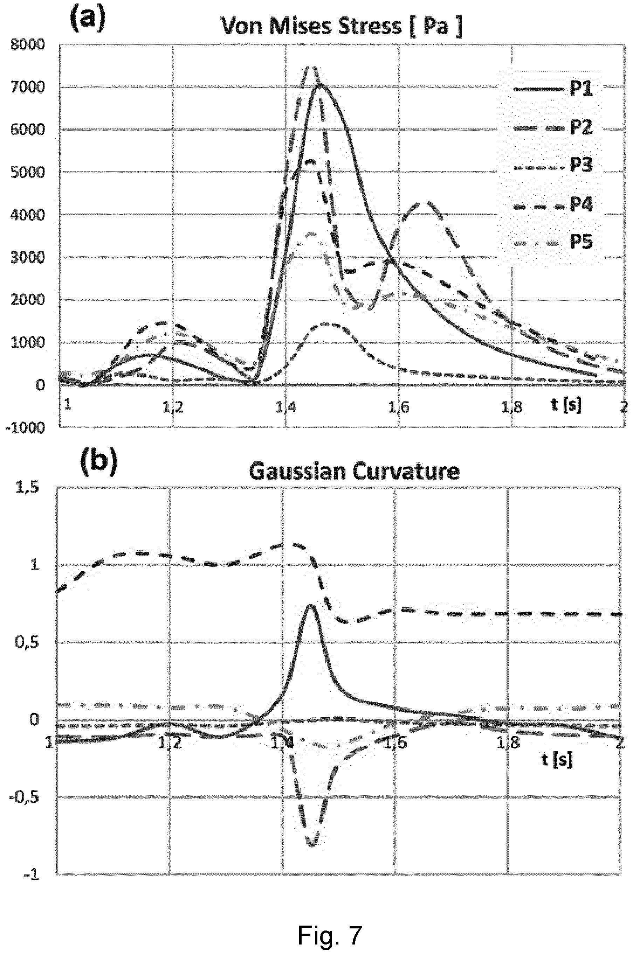

Fig. 7 shows stress distribution and Gauss curvature over time at five measuring points P1-P5.

In the tested model, the greatest curvature is observed in the front and middle parts of the plaque. In the front part of the plaque the maximum angle of inclination between the tangent curve and the axis of the vessel (stream ) is also observed. The greater this angle, the more the outer plaque surface is inclined towards the main flow direction. For a 90-degree angle, the wall is a perpendicular obstacle perpendicular to the stream.

Since the force of the jet thrust on this obstacle depends both on the angle of incidence of the jet and on the flow velocity, the maximum force values lie between the areas of the maximum inclination of the wall and the area of maximum velocity of the flow directly adjacent to the wall.

The negative value of the thrust force appears in the rear part of the lamina, even before the area where the backflow appears as a result of the stream detachment. It is mainly caused by the negative value of the kata. Also for this reason, the negative value of the thrust force is visible in the initial part mainly along the side curves c2 and c3. The maximum positive force value appears at the site of maximum reverse flow

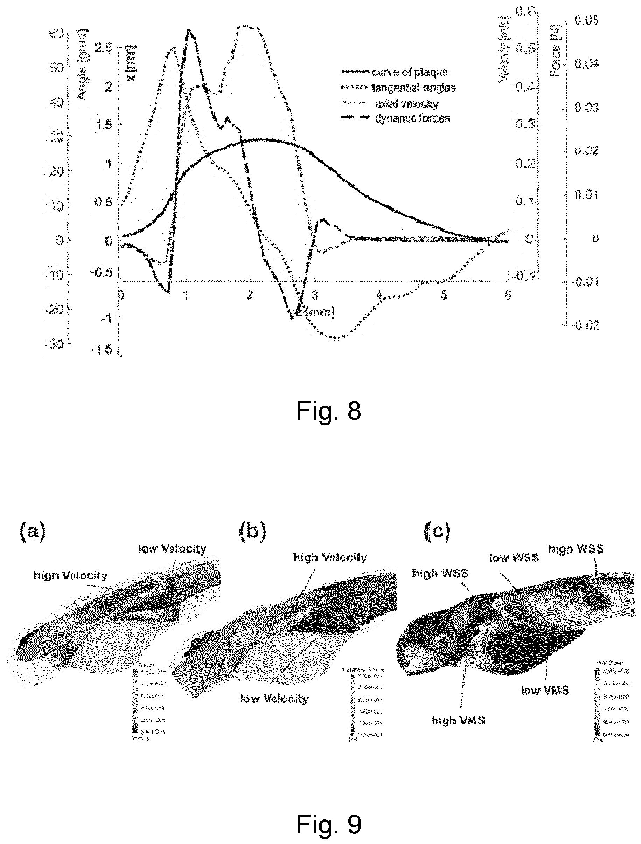

Fig. 8 shows influence of curvature, angle of inclination of a tangent curve to axis of vessel on the axial flow and pressure force, measured along the C1 curves.

The numerical simulation shown in Fig. 9 (wherein plot (a) illustrates velocity, plot (b) illustrates Von Misses Stress and plot (c) illustrates wall shear) shows that the greatest pressure force acts in the front part of the plate where the alpha angle is the greatest. In the top part of the lamina this force is minimal - a similar distribution is observed for the VMS.

A soft plaque deforms more easily, while a harder plaque, causes a greater stress inside cap layer.

The high shear stress, endothelial damage and arterial inflammation initiate the formation of early atherosclerotic plaques. In atherosclerotic arteries, proximal plaques regions are mainly exposed to high shear stresses and in distal section on the low shear stresses.

In the initial phase of the cycle, the maximum curvature is observed at the base of the lamina and in the center, at its highest point, while in the middle phase of the cycle there is also a large curvature in the front part of the plaque near place of its greatest deformation.

Non-invasive CT / MRI direct measurement of curvature on small plaques is very inaccurate due to the low resolution of medical images. However, modeling techniques, along with reconstruction 3D and numerical analysis of curvature changes, can significantly reduce this limitation. In this way, Local Gaussian Curvatures could become a new marker to facilitate the diagnosis of stress change occurring in both artery and plaque.

In addition, dynamic pressure (RDP) analysis along with the degree of constriction (stenosis) (DS) can help assess the probability of rupture or damage to the plaque (as shown in Fig. 10).

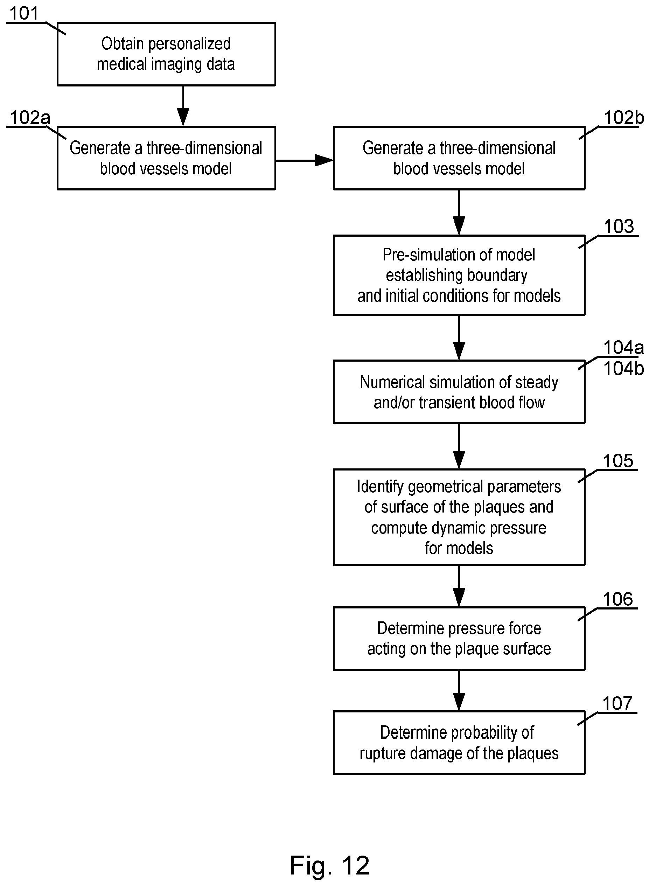

The conclusions related to the phenomena described above can be effectively used in modeling blood vessels and determining influence of biomechanical forces on deformation and stresses of arthrosclerosis plaque, in particular to predict probabilities of rupture or damage to the atherosclerotic plaque, by a method shown in Fig. 12.

In step 101, medical imaging data of the blood vessels is obtained.

Next, in step 102a, a personalized three-dimensional model of blood vessels is generated, such as shown in Fig. 4(a). When preparing the model, one or more pathological plaques are identified on the image of coronary vessels and their location and shape is included in the model. Subsequently, in the step 102b the three-dimensional reconstructed model of blood vessels is generated, that reflects the condition of healthy blood vessels and lacks lesions visible in the results of medical imaging - in tomography.

Next, a volumetric calculation grid is generated for the blood vessels model in step 103, for which the equation of blood flow dynamics is solved in each node to determine local velocity and pressure values.

Preparing the numerical model and establishing initial boundary condition, both for steady end transient flow, can be performed in step 104a and, under high-intensity physical exercise conditions. The definition of these conditions is explained below.

The publication of Doge et al. in "Lumen diameter of normal human coronary arteries. Influence of age, sex, anatomic variation, and left ventricular hypertrophy or dilation." Circulation. 86:232-246. 1992, presents a reconstructed normal data set against which to compare lumen dimensions in various pathological states. It indicates that the precise knowledge of the expected "normal" lumen diameter at a given coronary anatomic location may have the particular value in the investigation of estimate of coronary disease severity.

Based on this publication it can be assumed that the maximal flow under high effort will be about 5 times higher than the average flow in the coronary artery statistically average diameter of 4.5 mm measured in the middle left main artery of right coronary artery-dominant in normal men, under resting condition.

The determination of patient specific flow rate can be estimated based on flow-mass relationships obtained from morphometric studies, presented by Choy JS in "Scaling of Myocardial Mass to Flow and Morphometry of Coronary Arteries", J Appl Physiol. 104(5): 1281-1286. 2008.

According to this studies the maximal coronary inlet flow rate can be written as:

$$Qin = \beta D^n$$

wherein:

β - correlation coefficient
D- diameter of inlet coronary artery
n- power number, in our example n=2.

In step 104a, numerical simulation of transient blood flow is performed for linearly increased flow rate (from a laminar flow to a developed turbulent flow) and pre-established initial conditions in the blood vessel model. Based on pressure and mass flow calculations, in the step 103, simultaneously on the inlet and on every outlet of the models the energy of blood flow is calculated for every time step of transient flow. Depending on necessity, the blood flow conditions on the inlet to the model may vary significantly between individual tests. The numerical flow simulation is carried out for systolic pressure and inlet flow rate with values corresponding to high exertion conditions (as described by Wolthuis RA et al, in "The response of healthy men to treadmill exercise". Circulation. 55: 153-157. 1977 and Fletcher GF et al, in "Exercise Standards for Testing and Training. A Statement for Healthcare Professionals From the American Heart Association". Circulation. 104:1694-1740. 2001). According to these data an average systolic pressure of test can be determined over a wide pressure range under high exertion conditions (PI on Fig. 1,7).

In step 104b, in comparison to the simulation performed in step 104a, the simulation may be performed for a steady flow (for laminar as well developed turbulent flow) under fixed value of inlet pressure and outlets flow rate conditions.

Using numerical calculations for steady flow instead of calculations for transient flow will shorten the time needed to obtain test results from several hours to several minutes. It is extremely important from the point of view of the diagnosis of coronary vessels and the quick selection of the treatment procedure.

In step 105, for a particular plaque to be examined (step 105 and the following steps can be performed again for other plaques), the plaque geometrical parameters are identified, such as a shape, curvature, curvature of the major surface, and/or Gauss curvature of the plaque surface, as well as there are computed average cross-section of the vessel and dynamic pressure in region of plaques for stenosed and reconstructed models.

Then, in step 106, a pressure force acting on the plaque surface at any one or more points is determined as force acting on a fixed curved wall of the atherosclerotic plaque, calculated from the formula:

$$F = \sqrt{F_T^2 + F_N^2} = 2\rho QU sin\frac{\alpha}{2}.$$

wherein:

$\alpha$ is the angle of the slope between the tangent curve at the pressure point and the main axis of the vessel;
$F_T$ and $F_N$ are tangent and normal components of force to the surface of the plaque at the point of action.

In step 107, the method includes determining the likelihood of wafer rupture or failure by comparing the compressive force present in the wafer to a reference compressive force. For example, this can be done by comparing with the force acting on a specific stationary obstacle, by determining the parameters RDP (Relative Dynamic Pressure) and DS (Degree of Stenosis).

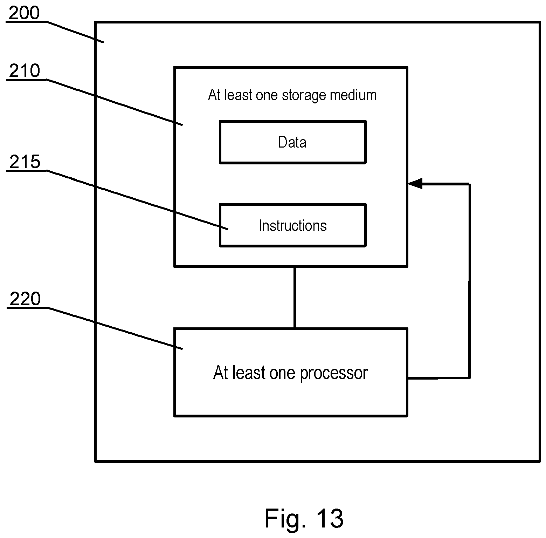

The functionality described herein can be implemented in a computer system 200, such as shown in Fig. 13. The system 200 may include at least one nontransitory processor-readable storage medium 210 that stores at least one of processor-executable instructions 215 or data; and at least one processor 220 communicably coupled to the at least one nontransitory processor-readable storage medium 210. The at least one processor 220 may be configured to (by executing the instructions 215) perform the methods presented herein, in particular the method presented in Fig. 12.

While the invention has been described with respect to a limited number of embodiments, it will be appreciated that many variations, modifications and other applications of the invention may be made. Therefore, the claimed invention as recited in the claims that follow is not limited to the embodiments described herein.

REFERENCES

The following references have been cited in the description:

- Abdelali M, Reiter S, Mongrain R, Bertrand M, L'Allier PL, Kritikou EA, Tardif JC. 2014. Cap buckling as a potentia lmechanism of atherosclerotic plaque vulnerability. JMech Behav BiomedMater. 32:210-24. 2014.
- Adam J. Brown et al. 2016. Role of biomechanical forces in the natural history of coronary atherosclerosis. Nat Rev Cardiol. 13(4):210-20. 2016.
- Bentzon J.B., , Otsuka F., Virmani R., Falk E. 2014. Mechanisms of Plaque Formation and Rupture. Circ Res. 114:1852-1866. 2014.
- Bentzon JF, Otsuka F, Virmani,R, Falk E. 2014. Mechanisms of Plaque Formation and Rupture. Circ Res. 114:1852-1866. 2014.
- Brenda R.Kwak et al. 2014. Biomechanical factors in atherosclerosis:mechanisms and clinical implications. European Heart Journal. 35, pp. 3013-3020. 2014.
- C.Akyildiz, LambertSpeelman,FrankJ.H.Gijsen. 2014. Mechanical properties of human atherosclerotic intima tissue. Journal ofBiomechanics. 4; 773-783. 2014.
- Cheng GC, Loree HM, Kamm RD, Fishbein MC, Lee RT. 1993. Distribution of circumferential stress in ruptured and stable atherosclerotic structural analysis with histopathological correlation. Circulation. 87:1179-1187. 1993.
- Choi G., et al. 2015. Coronary Artery Axial Plaque Stress and its Relationship With Lesion Geometry. JACC: Cardiovascular Imaging. 8;10;1156-1166. 2015.

- Costopoulos C, Timmins LH, Huang Y, Hung OY, et al. 2019. Impact of combined plaque structural stress and wall shear stress on coronary plaque progression, regression, and changes in composition. Eur Heart J. 7;40(18):1411-1422. doi: 10.1093/eurheartj/ehz132. PMID: 30907406; PMCID: PMC6503452. 2019.
- Doriot PA, Dorsaz PA, Dorsaz L, De Benedetti E, Chatelain P, Delafontaine P. 2000. In-vivo measurements of wall shear stress in human coronary arteries. Coron Artery Dis. 11(6):495-502. 2000.
- Finet, G., Ohayon, J., Rioufol, G., Lefloch, S., Tracqui, P., Dubreuil,. 2007. Morphological and biomechanical aspects of vulnerable coronary plaque. Archives des maladies du coeur et des vaisseaux 100 (6-7), 547-553. 2007.
- Jones, Robert M. 2009. Yielding and Yield Criteria. Deformation Theory of Plasticity, p. 151-158, Section 4. s.l. : Bul Ridge Publishing, 2009.
- Kwak BR, Bäck M, Bochaton-Piallat ML, Caligiuri G, Daemen MJ, Davies PF, Hoefer IE, Holvoet P, Jo H, Krams R, Lehoux S, Monaco C, Steffens S, Virmani R, Weber C, Wentzel JJ, Evans PC. 2014. Biomechanical factors in atherosclerosis: mechanisms and clinical implications. Eur Heart J. 14;35(43):3013-20, 3020a-3020d. doi: 10.1093/eurheartj/ehu353. Epub 2014 Sep 17. PMID: 25230814; PMCID: PMC4810806. 2014.
- Lee RT, Grodzinsky AJ, Frank EH, Kamm RD, Schoen FJ. 1991. Structure dependent dynamic mechanical behavior of fibrous caps from human atherosclerotic plaques. Circulation; 83:1764-770. 1991.
- Loree HM, Kamm RD, Stringfellow RG, Lee RT. 1992. Effects of fibrous cap thickness on peak circumferential stress in model atherosclerotic vessels. Circ Res. 71:850-858. 1992.
- Malek AM, Alper SL, Izumo S. 1999. Hemodynamic shear stress and its role in atherosclerosis. JAMA. 1;282(21):2035-42. 1999.
- Malota, Z., Glowacki, J., Sadowski, W. et al. 2018. Numerical analysis of the impact of flow rate, heart rate, vessel geometry, and degree of stenosis on coronary hemodynamic indices. BMC Cardiovasc Disord 18, 132 (2018). https://doi.org/10.1186/s12872-018-0865-6 2018.
- Menter, F.R. 1994. Two-equation eddy-viscosity turbulence models for engineering applications. AIAA-Journal ;32(8),1598 - 1605. 1994.
- Mises., Richard Elder von. 1913. Mechanik der festen Körper im plastisch deformablen Zustand. Gottin. Nachr. Math. Phys., vol. 1, pp. 582-592. 1913.
- Seneviratne A., Hulsmans M.,Holvoet P., Monaco C. 2013. Biomechanical factors and macrophages in plaque stability. Cardiovascular Research. 99; 2,15;284-293. 2013.
- Thondapu V, et.al. 2017. Biomechanical stress in coronary atherosclerosis: emerging insights from computational modelling. European Heart Journal (2017) 38, 81-92. 2017.
- Vikas Thondapu et al. 2017. Biomechanical stress in coronary atherosclerosis:emerging insights from computational modelling. European Heart Journal. 38, 81-92. 2017.

**Claims**

1. A computer-implemented method for modelling blood vessels, the method comprising the steps of:

   - obtaining (101) medical imaging data of the blood vessels;
   - generating (102a) a three-dimensional model of the blood vessels, based on the medical imaging data including identifying one or more pathological plaques;
   - generating (102b) a three-dimensional reconstructed model of the blood vessels that reflects the condition of healthy blood vessels and lacks lesions visible in the results of medical imaging;
   - performing pre-simulation (103) of the three-dimensional model, establishing boundary conditions and initial conditions for both models for a steady flow of blood and a transient flow of blood;
   - performing (104a) a numerical simulation of the transient flow of blood, for selected physical and boundary conditions, for the three-dimensional model for an increasing blood flow rate that increases from a laminar flow to a developed turbulent flow, the simulation comprising, determined during the pre-simulation (103), initial conditions of blood flow;
   - performing (104b) a numerical simulation of the steady flow of blood, for selected physical and boundary conditions, for the three-dimensional model for transitional or turbulent flow, the simulation comprising, determined during the pre-simulation (103), initial conditions of blood flow; and
   - for a selected plaque, identifying (105) geometrical parameters of a surface of the plaque, including shape, curvature, curvature of the major surface, and/or Gauss curvature of the plaque surface.

2. The method of claim 1, comprising determining (106) a pressure force acting on the plaque surface at any one or more points as a force acting on a fixed curved wall of the atherosclerotic plaque for a steady flow and/or for a transient flow.

3. The method of claim 2, comprising determining (106) a pressure force acting on the plaque surface at any one or more points as a force acting on a fixed curved wall of the atherosclerotic plaque by means of a formula:

$$F = \sqrt{F_T^2 + F_N^2} = 2\rho Q U sin\frac{\alpha}{2}.$$

wherein:

$\alpha$ is the angle of the slope between the tangent curve at the pressure point and the main axis of the vessel;
$F_T$ and $F_N$ are tangent and normal components of force to the surface of the plaque at the point of action;

4. The method of any of previous claims, further comprising determining (107) a probability of rupture or damage to the plaque, by comparing the pressure force present in the plaque with a reference pressure force for normal, healthy coronary vessels, based on a calculated Reference Dynamic Pressure (RDP) and Degree of Stenosis (DS).

5. The method of claim 4, wherein the Reference Dynamic Pressure (RDP) expresses the ratio of the dynamic pressure in the stenosed model to the dynamic pressure in the reference model in the plaques region and the Degree of Stenosis (DS) is a coefficient that increases as a function of the degree of vessel constriction.

6. A computer-implemented system, comprising:

- at least one nontransitory processor-readable storage medium (210) that stores at least one of processor-executable instructions or data; and
- at least one processor (220) communicably coupled to at least one nontransitory processor-readable storage medium, wherein at least one processor is configured to perform the steps of the method of any of the previous claims.

Fig. 1

Fig. 2

Fig. 3

Fig. 4

Fig. 5

Fig. 6

Fig. 7

Fig. 8

Fig. 9

Fig. 10

cenerline

DS = 0.75, RDP=0.501

Dynamic Pressure

stenosed

reconstructed

Fig. 11

101 — Obtain personalized
medical imaging data

102a — Generate a three-dimensional
blood vessels model

102b — Generate a three-dimensional
blood vessels model

103 — Pre-simulation of model
establishing boundary
and initial conditions for models

104a
104b — Numerical simulation of steady
and/or transient blood flow

105 — Identify geometrical parameters
of surface of the plaques and
compute dynamic pressure
for models

106 — Determine pressure force
acting on the plaque surface

107 — Determine probability of
rupture damage of the plaques

Fig. 12

200

210

215

220

At least one storage medium

Data

Instructions

At least one processor

Fig. 13

Europäisches
Patentamt

European
Patent Office

Office européen
des brevets

# EUROPEAN SEARCH REPORT

Application Number

EP 22 19 8895

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZBIGNIEW MALOTA ET AL: "Numerical analysis of the impact of flow rate, heart rate, vessel geometry, and degree of stenosis on coronary hemodynamic indices", BMC CARDIOVASCULAR DISORDERS, vol. 18, no. 1, 28 June 2018 (2018-06-28), XP055647778, DOI: 10.1186/s12872-018-0865-6 | 1-6 | INV. G16H30/40 G16H50/50 |
| Y | * page 2 – page 3 * <br> * page 9 * <br> * page 13 – page 14 * <br> * figure 1 * | 1-6 | |
| X | DALIN TANG ET AL: "Local Maximal Stress Hypothesis and Computational Plaque Vulnerability Index for Atherosclerotic Plaque Assessment", ANNALS OF BIOMEDICAL ENGINEERING, KLUWER ACADEMIC PUBLISHERS-PLENUM PUBLISHERS, NE, vol. 33, no. 12, 1 December 2005 (2005-12-01), pages 1789-1801, XP019272880, ISSN: 1573-9686 | 1-6 | |
| Y | * page 1790 – page 1791 * | 1-6 | TECHNICAL FIELDS SEARCHED (IPC) <br><br> G16H |
| A | CARDOSO LUIS ET AL: "Changing Views of the Biomechanics of Vulnerable Plaque Rupture: A Review", ANNALS OF BIOMEDICAL ENGINEERING, [Online] vol. 42, no. 2, 11 July 2013 (2013-07-11), pages 415-431, XP93028803, New York ISSN: 0090-6964, DOI: 10.1007/s10439-013-0855-x Retrieved from the Internet: URL:http://link.springer.com/article/10.10 07/s10439-013-0855-x/fulltext.html> [retrieved on 2023-03-03] * the whole document * | 1-6 | |

The present search report has been drawn up for all claims

| Place of search | Date of completion of the search | Examiner |
|---|---|---|
| Munich | 8 March 2023 | Rivera Farina, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DOGE et al.** Lumen diameter of normal human coronary arteries. Influence of age, sex, anatomic variation, and left ventricular hypertrophy or dilation. *Circulation,* 1992, vol. 86, 232-246
- **CHOY JS.** Scaling of Myocardial Mass to Flow and Morphometry of Coronary Arteries. *J Appl Physiol.,* 2008, vol. 104 (5), 1281-1286
- **WOLTHUIS RA et al.** The response of healthy men to treadmill exercise. *Circulation,* 1977, vol. 55, 153-157
- **FLETCHER GF et al.** Exercise Standards for Testing and Training. A Statement for Healthcare Professionals From the American Heart Association. *Circulation,* 2001, vol. 104, 1694-1740
- **ABDELALI M ; REITER S ; MONGRAIN R ; BERTRAND M ; L'ALLIER PL ; KRITIKOU EA ; TARDIF JC.** Cap buckling as a potentia lmechanism of atherosclerotic plaque vulnerability. *JMech Behav BiomedMater,* 2014, vol. 32, 210-24
- **ADAM J. BROWN et al.** Role of biomechanical forces in the natural history of coronary atherosclerosis. *Nat Rev Cardiol.,* 2016, vol. 13 (4), 210-20
- **BENTZON J.B. ; OTSUKA F. ; VIRMANI R. ; FALK E.** Mechanisms of Plaque Formation and Rupture. *Circ Res.,* 2014, vol. 114, 1852-1866
- **BENTZON JF ; OTSUKA F ; VIRMANI,R ; FALK E.** Mechanisms of Plaque Formation and Rupture. *Circ Res.,* 2014, vol. 114, 1852-1866
- **BRENDA R.KWAK et al.** Biomechanical factors in atherosclerosis:mechanisms and clinical implications. *European Heart Journal,* 2014, vol. 35, 3013-3020
- **C.AKYILDIZ, LAMBERTSPEELMAN ; FRANKJ.H.GIJSEN.** Mechanical properties of human atherosclerotic intima tissue. *Journal ofBiomechanics,* 2014, vol. 4, 773-783
- **CHENG GC ; LOREE HM ; KAMM RD ; FISHBEIN MC ; LEE RT.** Distribution of circumferential stress in ruptured and stable atherosclerotic structural analysis with histopathological correlation. *Circulation,* 1993, vol. 87, 1179-1187
- **CHOI G. et al.** Coronary Artery Axial Plaque Stress and its Relationship With Lesion Geometry. *JACC: Cardiovascular Imaging,* 2015, vol. 8 (10), 1156-1166

- **COSTOPOULOS C ; TIMMINS LH ; HUANG Y ; HUNG OY et al.** Impact of combined plaque structural stress and wall shear stress on coronary plaque progression, regression, and changes in composition. *Eur Heart J.,* 2019, vol. 40 (18), 1411-1422
- **DORIOT PA ; DORSAZ PA ; DORSAZ L ; DE BENEDETTI E ; CHATELAIN P ; DELAFONTAINE P.** In-vivo measurements of wall shear stress in human coronary arteries. *Coron Artery Dis.,* 2000, vol. 11 (6), 495-502
- **FINET, G. ; OHAYON, J. ; RIOUFOL, G. ; LEFLOCH, S. ; TRACQUI, P. ; DUBREUIL,.** Morphological and biomechanical aspects of vulnerable coronary plaque. *Archives des maladies du coeur et des vaisseaux,* 2007, vol. 100 (6-7), 547-553
- Yielding and Yield Criteria. **JONES, ROBERT M.** Deformation Theory of Plasticity. Bul Ridge Publishing, 2009, 151-158
- **KWAK BR ; BÄCK M ; BOCHATON-PIALLAT ML ; CALIGIURI G ; DAEMEN MJ ; DAVIES PF ; HOEFER IE ; HOLVOET P ; JO H ; KRAMS R.** Biomechanical factors in atherosclerosis: mechanisms and clinical implications. *Eur Heart J.,* 17 September 2014, vol. 35 (43), 3013-20
- **LEE RT ; GRODZINSKY AJ ; FRANK EH ; KAMM RD ; SCHOEN FJ.** Structure dependent dynamic mechanical behavior of fibrous caps from human atherosclerotic plaques. *Circulation,* 1991, vol. 83, 1764-770
- **LOREE HM ; KAMM RD ; STRINGFELLOW RG ; LEE RT.** Effects of fibrous cap thickness on peak circumferential stress in model atherosclerotic vessels. *Circ Res.,* 1992, vol. 71, 850-858
- **MALEK AM ; ALPER SL ; IZUMO S.** Hemodynamic shear stress and its role in atherosclerosis. *JAMA,* 1999, vol. 282 (21), 2035-42
- **MALOTA, Z. ; GLOWACKI, J. ; SADOWSKI, W. et al.** Numerical analysis of the impact of flow rate, heart rate, vessel geometry, and degree of stenosis on coronary hemodynamic indices. *BMC Cardiovasc Disord,* 2018, vol. 18 (2018), 132
- **MENTER, F.R.** Two-equation eddy-viscosity turbulence models for engineering applications. *AIAA-Journal,* 1994, vol. 32 (8), 1598-1605
- **MISES., RICHARD ELDER VON.** Mechanik der festen Körper im plastisch deformablen Zustand. Gottin. Nachr. *Math. Phys.,* 1913, vol. 1, 582-592

- **SENEVIRATNE A. ; HULSMANS M. ; HOLVOET P. ; MONACO C.** Biomechanical factors and macrophages in plaque stability. *Cardiovascular Research,* 2013, vol. 99, 284-293
- **THONDAPU V.** Biomechanical stress in coronary atherosclerosis: emerging insights from computational modelling. *European Heart Journal,* 2017, vol. 38, 81-92

- **VIKAS THONDAPU et al.** Biomechanical stress in coronary atherosclerosis:emerging insights from computational modelling. *European Heart Journal,* 2017, vol. 38, 81-92